# EUROPEAN PATENT SPECIFICATION

(11) **EP 3 903 745 B1**
(45) Date of publication and mention of the grant of the patent: **19.04.2023**
(21) Application number: 19902238.5
(22) Date of filing: 05.11.2019
(51) Int. Cl.: A61F 2/962, A61F 2/966, A61F 2/95

(54) **DELIVERY SYSTEM FOR MEDICAL IMPLANT**
ABGABESYSTEM FÜR EIN MEDIZINISCHES IMPLANTAT
SYSTÈME D'ADMINISTRATION POUR IMPLANT MÉDICAL

(30) Priority: 29.12.2018 CN 201811647489
(43) Date of publication of application: 03.11.2021
(73) Proprietor: Shanghai Intervascular MedTech Co., Ltd., Shanghai 201318 (CN)
(72) Inventor: JI, Qingru, Shanghai 201318 (CN); LIU, Mengqin, Shanghai 201318 (CN); ZHU, Qing, Shanghai 201318 (CN); YUAN, Zhenyu, Shanghai 201318 (CN)
(74) Representative: Lavoix
(86) International application number: PCT/CN2019/115639
(87) International publication number: WO 2020/134593

(56) References cited:
- US-A1- 2005 027 305
- US-A1- 2014 257 459
- US-A1- 2014 277 340

## Description

### TECHNICAL FIELD

The present invention relates to the field of medical surgical instruments and, in particular, to a delivery system for a medical implant.

### BACKGROUND

At present, cardiovascular disease is the number one killer of human beings. Medical implants including stents have been widely used in the treatment of various vascular diseases (e.g., aneurysms, dissections, thrombosis, stenosis, etc.). Most stents are implanted into patients' blood vessels through minimally invasive intervention. In general, such a procedure involves: loading a stent into a delivery system; advancing the delivery system to a target site through a vascular access established with a guidewire/catheter; releasing the stent by manipulating a release means in the delivery system; and withdrawing the delivery system, leaving the stent in the patient's body.

The release of the stent must satisfy the following requirements. For a relatively long stent, it is necessary to minimize the time required for release and establish a blood flow as soon as possible in order to shorten a time period of ischemia of peripheral blood vessels or lower extremity blood vessels. Therefore, the release must be accomplished in a very short time over a long stroke length. Current delivery systems available on the market can rarely meet such performance requirements.

US 2014/277340 A1 describes a hemostasis valve.

US 2005/027305 A1 describes an integrated mechanical handle with quick slide mechanism.

US 2014/257459 A1 describes a stent delivery system.

### SUMMARY OF THE INVENTION

It is an objective of the present invention to provide a delivery system for a medical implant suitable for releasing stents of various lengths, in particular, those that are relatively long while requiring quick and accurate release.

This objective is attained by a delivery system according to claim 1.

Further embodiments of the delivery system are described in claims 2 to 14.

With the proposed delivery system for a medical implant, stents of various lengths can be released by rotating and/or axially retracting the release handle and thus driving distal movement of the rack. It allows a long release stroke length, and quick release can be achieved by axially retracting the release handle. In particular, quick and accurate release of long stents is made possible.

Additionally, the structural design of the proposed delivery system for a medical implant is simple but smart. The use of the honeycomb-like networks of handle reinforcing ribs not only results in greatly enhanced overall strength of the handle but can also achieve this at reasonable cost with a simple fabrication process. The delivery system is able to release long stents and provide an additional option for clinical physicians.

### BRIEF DESCRIPTION OF THE DRAWINGS

Fig. 1 is a structural schematic of a delivery system for a medical implant according to an embodiment of the present invention.
Fig. 2a schematically illustrates a release handle assembled with a handle holder according to an embodiment of the present invention.
Fig. 2b is a schematic cross-sectional view of Fig. 2a taken along AA according to an embodiment of the present invention.
Fig. 2c shows a schematic enlarged view of a portion of Fig. 2b according to an embodiment of the present invention.
Fig. 3 is a schematic diagram of a release switch according to an embodiment of the present invention.
Fig. 4a is a schematic three-dimensional view of a release handle and a release switch according to an embodiment of the present invention.
Fig. 4b is a schematic cross-sectional view of a release handle and a release switch according to an embodiment of the present invention.
Fig. 4c is a schematic cross-sectional view of the release handle of Fig. 4b in a released configuration taken along BB according to an embodiment of the present invention.
Fig. 4d is a schematic cross-sectional view of the release handle of Fig. 4b in a locked configuration taken along BB according to an embodiment of the present invention.
Fig. 4e is a schematic enlarged view of a portion of Fig. 4d according to an embodiment of the present invention.
Fig. 5 shows a schematic diagram of an upper handle shell according to an embodiment of the present invention.
Fig. 6a is a schematic diagram of a lower handle shell employing scattered reinforcing ribs according to an embodiment of the present invention.
Fig. 6b is a schematic diagram of a lower handle shell employing metal support bars according to another embodiment of the present invention.
Fig. 6c is a schematic diagram of a lower handle shell employing a honey-comb-like network of reinforcing ribs according to yet another embodiment of the present invention.
Fig. 7 schematically illustrates a lower handle shell assembled with a rack according to an embodiment of the present invention.
Fig. 8 is a schematic enlarged view of a portion of Fig. 7 according to an embodiment of the present invention.
Fig. 9 is a schematic cross-sectional view of a handle housing according to an embodiment of the present invention.
Fig. 10 is a schematic partial cross-sectional view of a delivery system for a medical implant according to an embodiment of the present invention.
Fig. 11 is a schematic cross-sectional view of a portion containing a stent according to an embodiment of the present invention.
Fig. 12 is a schematic cross-sectional view of a delivery system for a medical implant in an initial configuration according to an embodiment of the present invention.
Fig. 13 is a schematic cross-sectional view of a delivery system for a medical implant that has experienced a rotational release process according to an embodiment of the present invention.
Fig. 14 is a schematic cross-sectional view of a delivery system for a medical implant with a maximum release stroke length being reached according to an embodiment of the present invention.

In these figures:
101-release handle; 101a-limiting groove;
102-handle holder; 102b-ring-like support plate; 103-upper handle shell; 103a-upper handle shell reinforcing plate; 103b-upper handle shell reinforcing rib; 104-lower handle shell; 104a-lower handle shell reinforcing plate; 104b-reinforcing rib; 11-handle housing; 11a-step; 11b-tip section; 11c-first shaft section; 11d-second shaft section; 105-release switch; 105a-cam; 105b-rotation stopper; 105c-bridging leg; 106-handle end cover;
20-rack; 20a-tooth; 20b-rack internal cavity; 20c-rack slideway; 20d-rack reinforcing rib; 20e-rack reinforcing ribs end face;
301-inner tube; 302-spring; 303-stent; 304-outer sheath; 305-outer sheath connecting member; 306-rack connecting member; 307-sealing ring;
401-inner tube trailing end connecting member; 402-protective tube; 51-liquid supply tube; 52-one-way valve;
604-lower handle shell; 604a-lower handle shell reinforcing plate; 604b-reinforcing rib;
704-lower handle shell; 704a-lower handle shell reinforcing plate; 704b-metal support bar.

### DETAILED DESCRIPTION

The present invention will be described below in greater detail by way of specific examples with reference to the accompanying drawings. Features and advantages of the invention will be more apparent from the following description, and from the appended claims. Note that the figures are provided in a very simplified form not necessarily drawn to exact scale for the only purpose of helping to explain the disclosed exemplary embodiments in a more convenient and clearer way.

As used herein, the term "distal" is intended to refer to an end relatively located away from a target diseased site in a patient (where a stent is to be implanted) during a surgical procedure, while the term "proximal" is intended to refer to an end relatively close to the diseased site (the targeted stent implantation site).

Embodiments disclosed herein relate to delivery system for the medical implants capable of releasing stents of various lengths by a rotating operation and/or an axially retracting operation. They allow a relative long release stroke length, and quick release can be achieved by axially retracting a release handle. In particular, quick and accurate release of long stents is made possible.

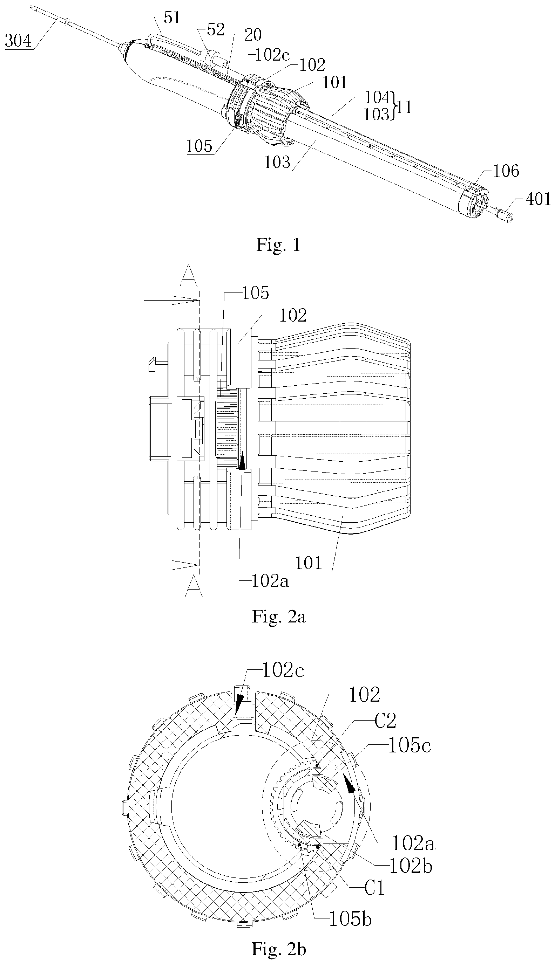

Fig. 1 schematically illustrates the structure of a delivery system for a medical implant according to an embodiment of the present invention. As shown in Fig. 1, the delivery system for the medical implant includes a handle housing 11, a release handle 101, a handle holder 102 and a rack 20. The handle housing 11 is a hollow tubular member defining a slot running along an axial direction of the hollow tubular member, and the rack 20 is received within the handle housing 11 in an orientation along the axial direction so as to be with at least some of the rack 20's teeth protruding out of the slot. The handle holder 102 is fixedly disposed over the handle housing 11, and the release handle 101 is disposed over the handle housing 11 into engagement with the teeth so that when the release handle 101 is rotated or axially retracted, the release handle 101 will drive the rack to move distally. When rotated, the release handle 101 engages against the handle holder 102 without axial movement relative thereto.

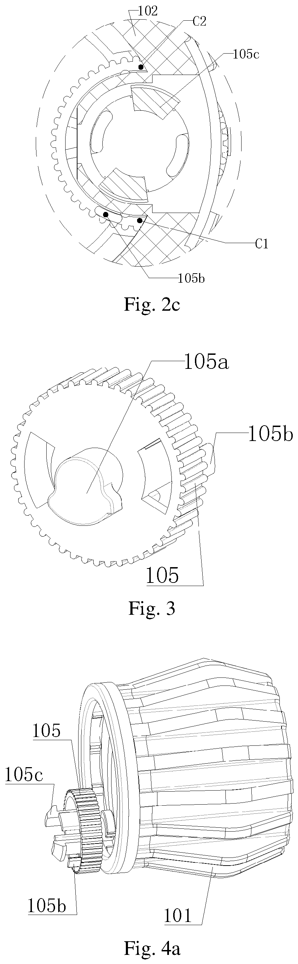

Fig. 2a schematically illustrates the release handle 101 assembled with the handle holder 102 according to this embodiment. Fig. 2b is a schematic cross-sectional view taken along AA in Fig. 2a according to this embodiment. Fig. 2c is a schematic enlarged view of a portion of Fig. 2b according to this embodiment. Fig. 3 is a schematic illustration of a release switch according to an embodiment of the present invention.

As shown in Figs. 2a, 2b, 2c and 3, a release switch 105 for limiting axial movement and circumferential rotation of the release handle 101 is disposed between the release handle 101 and the handle holder 102. This can prevent operational errors of the operator.

The release switch 105 may be in the form of, for example, a rotary disc, the release switch 105 is provided with a cam 105a at the distal end and a rotation stopper 105b at the proximal end, the rotation stopper 105b axially projecting from a location at a circumferential peripheral edge. On the side of the release switch 105 with the rotation stopper 105b, a number of bridging legs 105c may be spaced from one another along the circumference of a central circumferential portion of the release switch 105.

The handle holder 102 may define a first gap 102a for receiving the release switch 105 on a distal circumference. The release switch 105 may fit in the first gap 102a so that axes of the release switch 105 and the handle holder 102 are parallel to each other.

The handle holder 102 may be provided, around the first gap 102a, with a ring-like support plate 102b that is matched with the release switch 105. The ring-like support plate 102b may be provided at a plane that is perpendicular to the aforementioned axial direction. The bridging legs 105c on the release switch 105 may be engaged with the ring-like support plate 102b to enable stable and steady rotation of the release switch 105. An outer peripheral edge of the ring-like support plate 102b may be situated within a sector defined by an arc along which the rotation stopper 105b is moveable with the release switch 105. This means that the outer peripheral edge of the ring-like support plate 102b may not interfere with the rotation of the rotation stopper 105b. The outer peripheral edge of the ring-like support plate 102b may meet an inner wall of the handle holder 102 at a first notch C1 and a second notch C2. When the release switch 105 rotates clockwise or counterclockwise, the rotation stopper 105b will come into abutment against the wall at the first notch C1 or second notch C2, thus stopping the release switch and disallowing its further movement. A maximum permissible angle of rotation for the release switch defined by the first and second notches C1 and C2 may be 180° or less. In this way, the release switch 105 may be limited to rotation within an angle that is smaller than or equal to 180°.

With combined reference to Fig. 1, the ring-like handle holder 102 may be fixedly disposed over the handle housing 11 such that an axial second gap 102c in the handle holder 102 is aligned with the slot in the handle housing 11. The second gap 102c may radially extend through the handle holder 102 so that when the rack 20 moves in the handle housing 11 along the slot, its teeth protruding out of the slot can smoothly pass through the second gap in the handle holder 102.

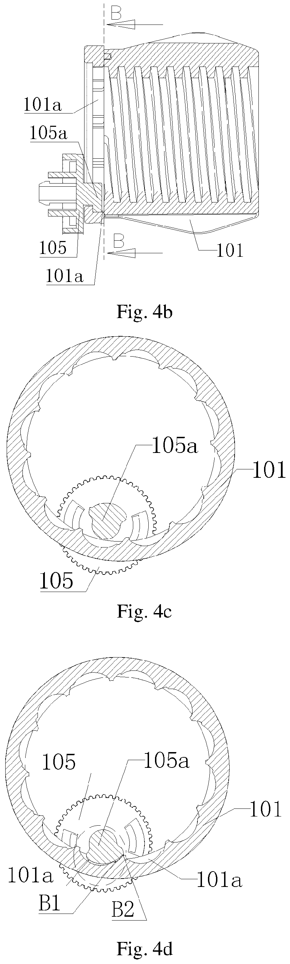

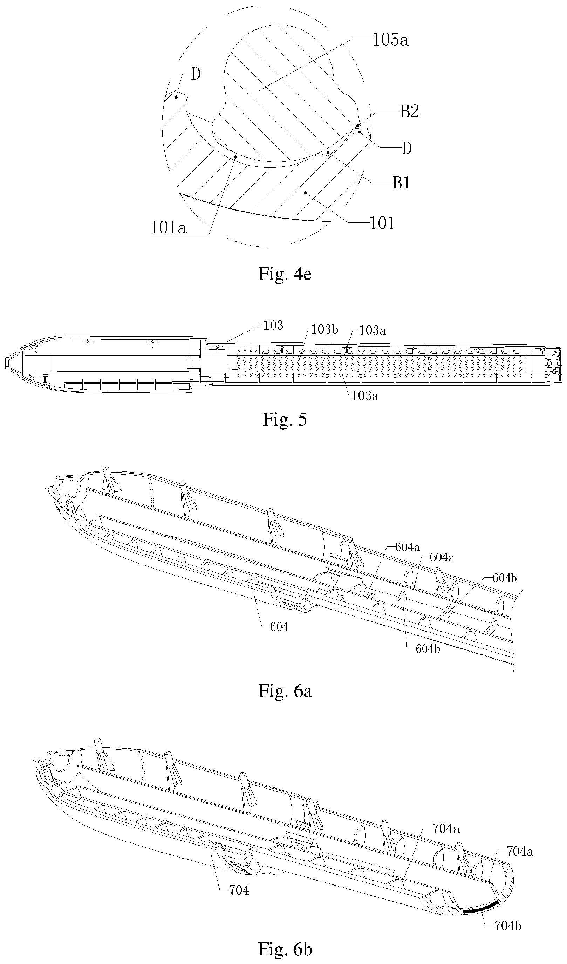

Fig. 4a shows a schematic three-dimensional view of the release handle 101 assembled with the release switch 105 according to this embodiment. Fig. 4b is a schematic cross-sectional view of the release handle 101 assembled with the release switch 105 according to this embodiment. Fig. 4c is a schematic cross-sectional view of the release handle 101 of Fig. 4b in a released configuration taken along BB according to this embodiment. Fig. 4d is a schematic cross-sectional view of the release handle 101 of Fig. 4b in a locked configuration taken along BB according to this embodiment. Fig. 4e is a schematic enlarged view of a portion of Fig. 4d according to this embodiment.

As shown in Figs. 4a, 4b, 4c, 4d and 4e, a number of limiting grooves 101a may be evenly provided along an inner circumference at a proximal end of the release handle 101, which are each complementary in shape with the cam 105a on the release switch 105 and contiguous with one another side by side. Each of the limiting grooves 101a has circumferential limiting protrusions D at its circumferentially opposing sides, as shown in Fig. 4d. When the release switch 105 is turned to engage the cam 105a with one of the limiting grooves 101a in the release handle 101, the release handle 101 is axially limited. At the same time, the release handle 101 is also circumferentially limited (i.e., it cannot rotate anymore) because the cam 105a blocks movement of the circumferential limiting protrusions D of the notch with which it engages. As shown in Fig. 4c, when the release switch 105 is turned to disengage the cam 105a from the limiting groove 101a in the release handle 101, the release handle 101 is unlocked (released) both axially and circumferentially. Therefore, locking (axial and circumferential limiting) and release of the release handle 101 can be accomplishing by engaging the cam 105a with one of the limiting grooves 101a and disengaging it from the limiting groove 101a.

The cam 105a may define a unilateral limiting protrusion B2, and each limiting groove 101a may define, at a location near one of the circumferential limiting protrusions D, a third notch B1 that is engageable with the unilateral limiting protrusion B2. With the unilateral limiting protrusion B2 of the cam 105a being in engagement with any third notch B1, the release switch 105 can exert a clockwise turning force, and with combined reference to Fig. 2c, upon the rotation stopper 105b on the opposing side coming into engagement with the first limiting notch C1, further clockwise rotational movement of the release switch 105 is limited. In this configuration, turning the release switch 105 counterclockwise will disengage the cam 105a from the limiting groove 101a and enable the release switch 105 to be turned counterclockwise. Likewise, the counterclockwise rotational movement will be limited when the rotation stopper 105b on the opposite side of the release switch 105 comes into engagement with the second notch C2. Instead of acting clockwise, the release switch 105 may be alternatively designed to exert a counterclockwise turning force in a similar way. Therefore, the rotation stopper 105b on the opposite side of the release switch 105 can only move along the arc between the first and second notches C1, C2, permitting the release switch 105 to rotate within an angle (angular stroke length) of 180° or less. This design can solve the problem with the current practice that although the release switch 105 can achieve a target unlocking (releasing) position, for example, a clockwise 60° turn, the operator like a physician would be unaware of this, and if he/she chooses the opposite direction, then the unlocking (releasing) will now require a 300° turn. By means of the unilateral limiting protrusion B2 of the cam 105a that can engage with the third notch B1 of any limiting groove 101a as well as of the limiting ability of the rotation stopper 105b, the release handle 101 can be unlocked (released) always by turning the release switch 105 in one direction by an angle that is smaller than or equal to 180°. The shortening of the required angular stroke length can avoid unnecessary operational effort to be paid by, for example, the physician, and save his/her previous time.

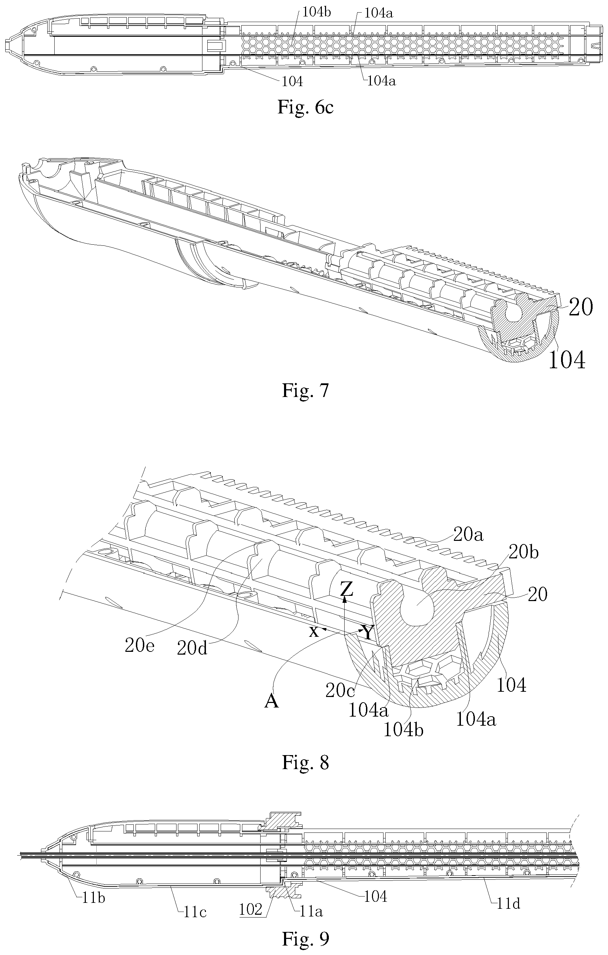

Fig. 5 shows a schematic diagram of an upper handle shell 103 according to this embodiment. Fig. 6a is a schematic diagram of a lower handle shell employing scattered reinforcing ribs 604b according to an embodiment of the present invention. Fig. 6b is a schematic diagram of a lower handle shell employing metal support bars 704b according to another embodiment of the present invention. Fig. 6c is a schematic diagram of a lower handle shell employing a honeycomb-like network of reinforcing ribs 104b according to yet another embodiment of the present invention. The handle housing 11 may include the upper and lower handle shells.

The following considerations are taken into account in the development of the upper and lower handle shells. In order to adapt to a very long release stroke length, the handle itself needs to be designed with a total length of, for example, 340-350 mm. This may lead to the following two challenges for using polymer injection molded parts as the components. One of the challenges is that the parts are prone to warpage during injection molding. The other challenge is that the handle assembled from such parts tends to deform under significant stress or exhibit poor bending or torsion resistance. Various attempts were made to resolve these difficulties. Since the upper and lower handle shells are structured identically, only the lower handle shell is detailed below as an example. As shown in Fig. 6a, the first attempt used a reinforcing plate 604a and scattered internal reinforcing ribs 604b. However, the physical prototype was found to be with limited torsion resistance. As shown in Fig. 6b, the later second attempt used a reinforcing plate 704a and metal support bars 704b. The metal support bars 704b were added during injection molding of the part for the lower handle shell 704, and this was found to be very difficult and costly. The third attempt used expensive glass fiber reinforced injection molding pellets.

Based on in-depth analysis of the above experimental attempts, we decided to adopt a handle reinforcing design with a honeycomb-like structure, as shown in Fig. 5 and Fig. 6.

As shown in Fig. 5, within the upper handle shell 103, two upper handle shell reinforcing plates 103a are arranged in a symmetrical configuration along the axial direction perpendicular to a reference plane. The reference plane is defined as a plane that passes through the lowest point of the lower handle shell 104 (in an orientation with the opening of this semi-tubular structure facing upward) and is tangent to the lower handle shell 104. In particular, the upper handle shell reinforcing plates 103a may extend axially from one end of the upper handle shell 103 to the other. Within the upper handle shell 103, there are also arranged upper handle shell reinforcing ribs 103b, the upper handle shell reinforcing plates 103a are attached to the upper handle shell reinforcing ribs 103b. Specifically, the upper handle shell reinforcing ribs 103b may make up a honeycomb-like network.

As shown in Fig. 6c, within the lower handle shell 104, two lower handle shell reinforcing plates 104a are arranged in a symmetrical configuration along the axial direction perpendicular to the reference plane. In particular, the lower handle shell reinforcing plates 104a may extend axially from one end of the lower handle shell 104 to the other. Within the lower handle shell 104, there are also arranged lower handle shell reinforcing ribs 104b, the lower handle shell reinforcing plates 104a are attached to the lower handle shell reinforcing ribs 104b. Specifically, the lower handle shell reinforcing ribs 104b may make up a honeycomb-like network.

According to this embodiment, using the honeycomb-like networks of reinforcing ribs in the upper and lower handle shells 103, 104 is sensible because they can be easily injection-molded at a reasonable expense while imparting significantly improved axial stiffness to the upper and lower handle shells 103, 104, which ensures that the handle components will not bend or twist when heavily resisted during a release process.

Fig. 7 schematically illustrates the lower handle shell 104 assembled with the rack 20 according to this embodiment. Fig. 8 is a schematic enlarged view of a portion of Fig. 7 according to this embodiment.

As shown in Figs. 5 to 8, the handle housing 11 includes the upper and lower handle shells 103, 104 detachably attached to each other. The upper and lower handle shells 103, 104 are semi-tubular members.

For ease of understanding and description, as noted above, the reference plane is defined as a plane that passes through the lowest point of the lower handle shell 104 (in an orientation with the opening of this semi-tubular structure facing upward) and is tangent to the lower handle shell 104. Additionally, the axial direction of the handle housing 11 is defined as an X-direction, a direction that is within a plane parallel to the reference plane and perpendicular to the X-direction as a Y-direction, and a direction perpendicular to the reference plane as a Z-direction. Surfaces A of the upper and lower handle shells 103, 104 where they are put into contact with each other may be parallel to the reference plane.

The teeth 20a of the rack 20 may be arranged side by side along the axial direction and some of them protrude out of the aforesaid slot. The rack 20 may have a rack internal cavity 20b extending through the rack axially and two rack slideways 20c, which are arranged in symmetry with respect to the axial (X-) direction and each assume an L-shaped radial cross-section. Each of the L-shaped rack slideways 20c may be made up of an arm oriented in the Y-direction and another arm oriented in the Z-direction. A number of rack reinforcing ribs 20d may be provided on the rack 20, which are arranged along the axial direction side by side with spaces therebetween, and the plurality of rack reinforcing ribs 20d have coplanar end faces 20e, the plane on which the end faces 20e of the rack reinforcing ribs are located is parallel to the reference plane.

The rack slideways 20c may fit with the lower handle shell reinforcing plates 104a. Specifically, a portion of the rack 20 between the symmetrical rack slideways 20c may fit in a space between the symmetrical lower handle shell reinforcing plates 104a, with surfaces of the rack slideways 20c perpendicular to the reference plane fitting against surfaces of the lower handle shell reinforcing plates 104a perpendicular to the reference plane. This limits the rack 20 in the Y-direction.

In addition, surfaces of the rack slideways 20c parallel to the reference plane may fit against surfaces of the lower handle shell reinforcing plates 104a parallel to the reference plane, and the end faces 20e of the rack reinforcing ribs may abut against the upper handle shell reinforcing plates 103a. This further limits the rack 20 in the Z-direction.

Limiting the rack 20 in both the Y- and Z-directions by fitting the rack slideways 20c against the lower handle shell reinforcing plates 104a at their surfaces parallel and perpendicular to the reference plane and by bringing the end faces 20e of the rack reinforcing ribs into abutment against the upper handle shell reinforcing plates ensures stable axial movement (in the X-direction) of the rack 20 without dislodging from the lower handle shell 104 or upper handle shell 103 and imparts greater release stability to the delivery system.

The rack slideways 20c, the lower handle shell reinforcing plates 104a and the upper handle shell reinforcing plate 103a may together provide a release path in the handle assembly of the delivery system for the medical implant. The lower handle shell reinforcing plates 104a may axially extend from one to the other end of the lower handle shell 104, and the lower handle shell reinforcing plates 104a may be integrally attached to the lower handle shell reinforcing ribs 104b, and the upper handle shell reinforcing plates 103a may axially extend from one to the other end of the upper handle shell 103, and the upper handle shell reinforcing plates 103a may be integrally attached to the upper handle shell reinforcing ribs 103b. Therefore, the lower and upper handle shell reinforcing plates 104a, 103a function both to make contribution to the release path and to strengthen the corresponding lower and upper handle shells 104, 103. Likewise, the rack slideways 20c also strengthen the rack 20 while forming part of the release path. Thus, all the lower handle shell reinforcing plates 104a, the upper handle shell reinforcing plates 103a and the rack slideways 20c are bifunctional members that participate in the establishment of the release path and increase the strength of the corresponding components.

Fig. 9 is a schematic cross-sectional view of the handle housing according to this embodiment. As shown in Figs. 1, 8 and 9, the handle housing 11 may include a tip section 11b, a first shaft section 11c and a second shaft section 11d. The tip section 11b, the first shaft section 11c and the second shaft section 11d are connected in series in the axially direction of the handle housing 11. The handle holder 102 may be fixedly disposed around the junction of the first and second shaft sections 11c, 11d. In this embodiment, a diameter of the first shaft section 11c is greater than a diameter of the second shaft section 11d. In alternative embodiments, the diameter of the first shaft section 11c may be equal to or smaller than that of the second shaft section 11d. In this embodiment, the first and second shaft sections 11c, 11d defines a step 11a at their junction so that the handle holder 102 is fixedly disposed around the step 11a. In alternative embodiments, the junction of the first and second shaft sections 11c, 11d may be otherwise structured. The tip section 11b may resemble a tip portion of a pen. Each of the first and second shaft sections 11c, 11d may be a hollow tubular section defining an axially extending slot. The interiors of the first and second shaft sections 11c, 11d may communicate with each other. Upon the rack 20 reaching the second shaft section 11d, the teeth 20a protrude from the slot, so that the release handle 101 may come into engagement with the teeth 20a.

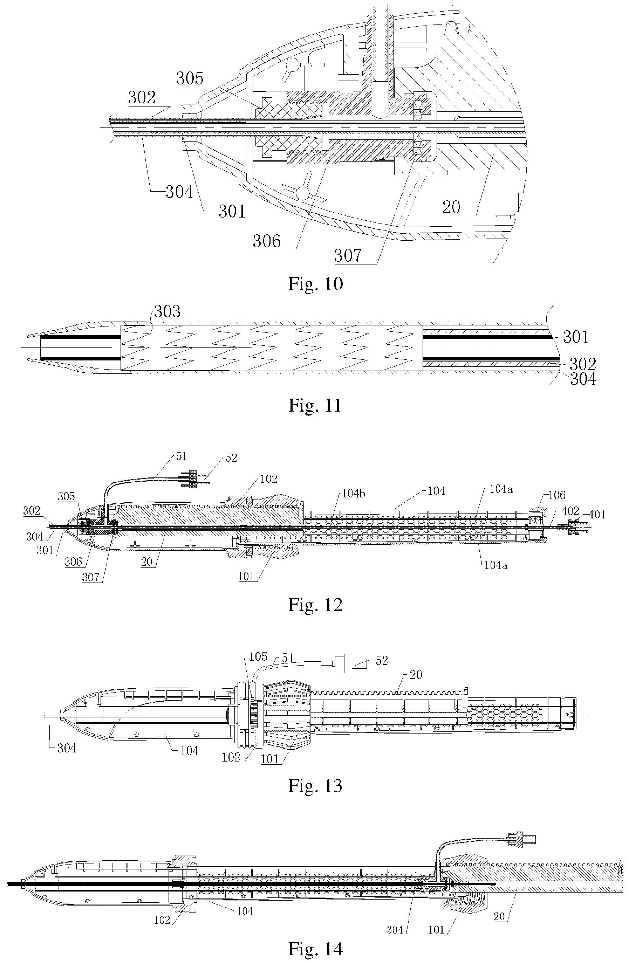

Fig. 10 is a schematic cross-sectional view of a portion of the delivery system for the medical implant according to this embodiment. Fig. 11 is a schematic cross-sectional view of a portion containing a stent according to this embodiment. As shown in Figs. 1, 10 and 11, in addition to the handle housing 11, the release handle 101, the handle holder 102 and the rack 20, the delivery system for the medical implant may further include an outer sheath 304. The handle housing 11 is a tubular member defining a slot running along an axial direction of the tubular member. The rack 20 is so received in the handle housing 11 that at least some teeth of the rack 20 protrude out of the slot. The handle holder 102 is fixedly disposed over the handle housing 11, and the release handle 101 is disposed over the handle housing 11 so as to engage the teeth. As such, the rack will distally move in response to the release handle 101 being rotated or axially pulled. When rotated, the release handle 101 engages with the handle holder 102 without any axial movement relative thereto. A distal end of the outer sheath 304 may be connected to a proximal end of the rack 20. With this arrangement, when turned or axially pulled, the release handle 101 will drive the rack 20 and hence the outer sheath 304 to move distally.

The delivery system for the medical implant may further include an inner tube 301. The outer sheath 304 may be disposed over the inner tube 301 and thus define an accommodating space with the inner tube 301. A stent 303 may be received in a proximal portion of the accommodating space, and the stent 303 is generally tubular. The stent may be uniformly crimped and loaded in the cavity between the inner tube 301 and the outer sheath 304.

With combined reference to Figs. 9 and 10, the proximal end of the rack 20 may be connected to the distal end of the outer sheath 304 within the tip section 11b along the axial direction. Specifically, an outer sheath connecting member 305 may be fixed to the distal end of the outer sheath 304, and a rack connecting member 306 may be arranged at the proximal end of the rack 20 with the aid of a sealing ring 307. The rack 20 may be fixedly connected to the outer sheath 304 by means of a threaded or snap connection between the rack connecting member 306 and the outer sheath connecting member 305. It will be appreciated that the fixed connection between the rack 20 and the outer sheath 304 may be accomplished by adhesive bonding, threads, snap fasteners or welding. Each of the outer sheath connecting member 305 and the rack connecting member 306 may define an axial through hole through which the inner tube 301 can be inserted. The rack internal cavity 20b of the rack 20 also allows the inner tube 301 to be axially inserted therethrough.

Fig. 12 is a schematic cross-sectional view of the delivery system for the medical implant according to this embodiment in an initial configuration. Fig. 13 is a schematic cross-sectional view of the delivery system for the medical implant according to this embodiment that has experienced a rotational release process. Fig. 14 is a schematic cross-sectional view of the delivery system for the medical implant according to this embodiment in a configuration with a maximum release stroke length being reached. As shown in Figs. 12 to 14, the delivery system for the medical implant according to this embodiment allows a maximum release stroke length of up to 280-300 mm, making it suitable for the release of stents for treating various vascular diseases, in particular those with a long diseased length or a complicated geometrical structure.

The delivery system for the medical implant according to this embodiment can release a stent in three distinct ways.

As shown Figs. 12, 1 and 10, in the initial configuration of the delivery system for the medical implant, the release handle 101 engages with the handle holder 102, with the release switch 105 limiting the release handle 101 axially and circumferential and with the rack 20 being distally aligned with the release handle 101.

In this configuration, with proximal portions of the handle holder 102 and the handle housing 11 being gripped, release can be achieved simply by rotating the release handle 101. Specifically, in case of the stent being a short one, after the release handle 101 is unlocked by rotating the release switch 105, it may be turned in an axially stationary way (i.e., with the release handle 101 engaging with the handle holder 102 and no axial movement). As a result, the release handle 101 transmits a driving force by means of the rack 20 that it engages, causing the outer sheath 304 to move with the rack 20 distally in the axial direction until the stent 303 is (exposed and) released. Alternatively, the release may also be accomplished simply by axially retracting the release handle 101. Specifically, in case of the stent 303 being a long one requiring quick release, after the release handle 101 is unlocked by turning the release switch 105 in the initial configuration, it may be directly gripped and quickly pulled distally. As a result, the rack 20 moves linearly and distally in synchronization with the release handle 101 along the axial direction, achieving quick release of the stent 303. Still alternatively, the stent may be also released by both rotating and axially retracting the release handle 101. Specifically, if the stent is too long to be fully exposed by simply axially pulling the release handle 101 backward, then after the release handle 101 is unlocked by turning the release switch 105 in the initial configuration, it may be turned in an axially stationary way (i.e., with the release handle 101 engaging with the handle holder 102 and no axial movement) until the rack 20 moves into proximal alignment with the release handle 101, as shown in Fig. 13. The release handle 101 may be then axially retracted distally, causing the rack 20 and hence the outer sheath 304 to move linearly and distally in synchronization with the release handle 101 in the axial direction, until the stent is released. As shown in Fig. 14, a maximum release stroke length will be reached when the proximal ends of the rack 20 and the release handle 101 are both aligned with a distal end of the handle housing 11. The maximum release stroke length may be, for example, 280-300 mm. It will be appreciated that the release stroke length is the axial distance travelled by the rack, i.e., the distance the outer sheath axially moves with the rack.

As shown in Figs. 11, 12 and 1, the axes of the handle housing 11, the outer sheath 304 and the inner tube 301 may be parallel to one another. Preferably, the axes of the handle housing 11, the outer sheath 304 and the inner tube 301 coincide with one another. The inner tube 301 may be axially inserted through the handle housing 11. Specifically, the inner tube 301 may be inserted sequentially through the through hole of the outer sheath connecting member 305, the through hole of the rack connecting member 306 and the rack internal cavity 20b of the rack 20 within the handle housing 11 and fixed to a distal end of the upper handle shell 103 by a protective tube 402. A handle end cover 106 may be fixed to a distal end of the handle housing 11. The inner tube 301 may axially extend so as to protrude out of the handle end cover 106 distally. An inner tube trailing end connecting member 401 may be detachably connected to a distal end of the protective tube 402, for example, by a threaded or tapered fit. As such, in case of the stent 303 being a long one requiring a large release stroke length, if it is insufficient to achieve the release even when the rack 20 has moved to the distal end of the handle housing 11, the inner tube trailing end connecting member 401 may be detached to expand the release stroke length and allow the rack to further move outside the handle housing 11 until the proximal end of the rack reaches the distal end of the handle housing.

The protective tube 402 may be disposed over the inner tube 301 and the proximal end of the protective tube 402 may be connected to a spring 302 within the tip section 11b. In this way, the inner tube 301, the spring 302, the protective tube 402 and the handle housing 11 are connected together. The inner tube 301 may be elongate, the portion of the inner tube 301 inside the handle housing 11 is supported and protected by the protective tube 402 and the portion of the inner tube 301 outside the handle housing 11 is supported and protected by the spring 302.

In addition to the portion extending across the whole length of handle housing 11, the inner tube 301 may further have an axial extension proximally extending outside the handle housing 11. The stent 303 may be crimped over a proximal portion of the extension, and the spring 302 may be disposed over a distal portion of the extension that is not encompassed by the stent 303. A distal portion of the spring 302 may be received within the tip section 11b, and the axially extending outer sheath 304 may be disposed over both the stent 303 and the spring 302. The outer sheath 304 may be proximally aligned with the inner tube 301 and distally fixed to the outer sheath connecting member 305. The outer sheath 304 may be provided to protect the stent 303 and configured to be able to move distally when driven by the rack 20. Since the inner tube 301 is fixed in the handle housing 11 and the stent 303 is crimped on the inner tube 301, as a result of the distal movement of the outer sheath 304 with the rack 20, the stent 303 can be exposed and released. A clearance between the outer sheath 304 and the inner tube 301 may accommodate stents 303 for treating various vascular diseases. The outer sheath 304 may be relatively long, and the inner tube 301 may be relatively thin. The spring 302 may be disposed in a distal portion of the clearance between the outer sheath 304 and the inner tube 301 that does not encompass the stent 303. This makes the structure within the outer sheath 304 more stable and more compliant.

A liquid supply tube 51 and a one-way valve 52 may be provided at a proximal end of the slot in the handle housing 11. The liquid supply tube 51 may be used to evacuate air from the delivery system for the medical implant by introducing physiological saline, and the one-way valve 52 may be configured to control the physiological saline to flow in only one direction.

In summary, with the delivery system for the medical implant of the present invention, stents of various lengths can be released by rotating and/or axially retracting the release handle and thus driving distal movement of the rack. This system allows a long release stroke length, and quick release can be achieved by axially retracting the release handle. In particular, quick and accurate release of long stents is made possible.

The following disclosure provides many different embodiments for implementing different features of the disclosure. Specific examples of components and arrangements are described below to simplify the present disclosure. These are, of course, merely examples and are not intended to limit the present invention. For example, in the following description, spatially relative terms, such as "under", "below", "lower", "upper" and the like, may be used for ease of description to describe one element or feature's relationship to another element(s) or feature(s) as illustrated in the figures. The spatially relative terms are intended to encompass different orientations of the device in use or operation in addition to the orientation depicted in the figures. The device may be otherwise oriented, for example, rotated 90 degrees or at other orientations, and the spatially relative descriptors used herein interpreted accordingly.

The embodiments disclosed herein are described in a progressive manner, with the description of each embodiment focusing on its differences from others. Reference can be made between the embodiments for their identical or similar parts. Since the system embodiments correspond to the method embodiments, they are described relatively briefly, and reference can be made to the method embodiments for details in the system embodiments.

## Claims

1. A delivery system for a medical implant, comprising a handle housing (11), a release handle (101), a handle holder (102) and a rack (20), the handle housing (11) implemented as a hollow tubular member defining a slot extending along an axial direction of the handle housing (11), the rack (20) received within the handle housing (11) in an orientation along the axial direction of the handle housing (11), at least some of teeth of the rack (20) protruding out of the slot, the handle holder (102) fixedly disposed over the handle housing (11), the release handle (101) disposed over the handle housing (11) into engagement with the teeth so that the rack (20) moves distally as a result of a rotation or axial retraction of the release handle (101), wherein when the release handle (101) is rotated, the release handle (101) engages with the handle holder (102) and stays stationary axially relative to the handle holder (102),
**characterized in that**:
a release switch (105) for limiting axial movement and circumferential rotation of the release handle (101) is arranged between the release handle (101) and the handle holder (102),
the release switch (105) has a distal end provided with a cam (105a), and an inner surface of a proximal end of the release handle (101) provided with a plurality of limiting grooves (101a) arranged circumferentially with respect to the release handle (101), each limiting groove (101a) engageable with the cam (105a) and having circumferential limiting protrusions on both ends along a circumferential direction.

2. The delivery system for a medical implant of claim 1, wherein the handle holder (102) has a distal end provided with a first gap, the release switch (105) partially embedded in the first gap and an axis of the release switch (105) being parallel to an axis of the handle housing (11).

3. The delivery system for a medical implant of claim 2, wherein the handle holder (102) is provided with a ring-like support plate (102b) matched with the release switch (105), the ring-like support plate (102b) having an axis parallel to the axis of the handle housing (11), and wherein the release switch (105) has a proximal end provided with at least two bridging legs (105c), each of which engages an inner surface of the ring-like support plate (102b).

4. The delivery system for a medical implant of claim 3, wherein an outer surface of the ring-like support plate (102b) meets an inner surface of the handle holder (102) at a first notch and a second notch, wherein the proximal end of the release switch (105) is provided with a rotation stopper (105b), and wherein when the release switch (105) is rotated clockwise or counterclockwise, the rotation stopper (105b) is rotated to the first notch or the second notch, the rotation stopper (105b) is engaged into the first notch or the second notch, thus defining a maximum permissible angle of rotation for the release switch (105).

5. The delivery system for a medical implant of claim 4, wherein the maximum permissible angle of rotation for the release switch (105) is smaller than or equal to 180°.

6. The delivery system for a medical implant of claim 1, wherein the cam (105a) is provided with a unilateral limiting protrusion, each of the plurality of limiting grooves (101a) provided with a third notch engageable with the unilateral limiting protrusion, the unilateral limiting protrusion and the third notch cooperate to enable unidirectional turning for the release switch (105).

7. The delivery system for a medical implant of claim 1, wherein the handle housing (11) comprises an upper handle shell (103) and a lower handle shell (104, 604, 704) that are detachably connected, and
wherein the upper handle shell (103) is provided therein with an upper handle shell reinforcing plate (103a) along an axial direction of the upper handle shell (103), wherein the lower handle shell (104, 604, 704) is provided therein with a lower handle shell reinforcing plate (104a, 604a, 704a) along an axial direction of the lower handle shell (104, 604, 704), wherein on a side of the rack (20) close to the lower handle shell (104, 604, 704) is provided with a rack slideway (20c) able to fit with the lower handle shell reinforcing plate (104a, 604a, 704a), the rack slideway (20c) extending along the axial direction of the handle housing (11), and wherein the rack (20) abuts against the upper handle shell reinforcing plate (103a) at a side thereof close to the upper handle shell (103).

8. The delivery system for a medical implant of claim 7, wherein a plurality of rack reinforcing ribs (20d) are provided on the rack (20) at intervals along an axial direction of the rack (20).

9. The delivery system for a medical implant of claim 7, wherein the upper handle shell (103) is further provided therein with a honeycomb-like network of upper handle shell reinforcing ribs (103b), and the lower handle shell (104, 604, 704) is further provided therein with a honeycomb-like network of lower handle shell reinforcing ribs (104b), and
wherein the upper handle shell reinforcing ribs (103b) are attached to the upper handle shell reinforcing plate (103a), and the lower handle shell reinforcing ribs (104b) are attached to the lower handle shell reinforcing plate (104a, 604a, 704a).

10. The delivery system for a medical implant of any one of claims 1 to 9, wherein the handle housing (11) comprises a tip section (11b), a first shaft section (11c) and a second shaft section (11d), which are connected in series in the axial direction, wherein the handle holder (102) is fixedly disposed around a junction of the first and second shaft sections.

11. The delivery system for a medical implant of claim 10, wherein a diameter of the first shaft section (11c) is greater than a diameter of the second shaft section (11d), a step (11a) provided at the junction of the first and second shaft sections, and the handle holder (102) fixedly disposed around the step (11a), and/or
wherein the tip section (11b) resembles a tip portion of a pen, and each of the first and second shaft sections (11c, 11d) is a hollow tubular member provided with the slot in the axial direction.

12. The delivery system for a medical implant of any one of claims 1 to 9, wherein the rack (20) defines a rack internal cavity (20b) extending through the rack (20) along the axial direction.

13. The delivery system for a medical implant of claim 2, wherein the handle holder (102) defines a second gap corresponding to the slot along the axial direction.

14. The delivery system for a medical implant of claim 1, further comprising an outer sheath (304) with a distal end connected to a proximal end of the rack (20), wherein rotating or axially retracting the release handle (101) causes distal movement of the rack (20) and hence distal movement of the outer sheath (304) with the release handle (101).

## Patentansprüche

1. Zuführungssystem für ein medizinisches Implantat, umfassend ein Griffgehäuse (11), einen Freigabegriff (101), einen Griffhalter (102) und eine Zahnstange (20) umfasst, wobei das Griffgehäuse (11) als hohles, rohrförmiges Element ausgeführt ist, das einen Schlitz definiert, der sich entlang einer axialen Richtung des Griffgehäuses (11) erstreckt, wobei die Zahnstange (20) innerhalb des Griffgehäuses (11) in einer Ausrichtung entlang der axialen Richtung des Griffgehäuses (11) aufgenommen ist, wobei zumindest einige der Zähne der Zahnstange (20) aus dem Schlitz hervorstehen, der Griffhalter (102) fest über dem Griffgehäuse (11) angeordnet ist, der Freigabegriff (101) in Eingriff mit den Zähnen über dem Griffgehäuse (11) angeordnet ist, sodass sich die Zahnstange (20) als Resultat einer Drehung oder eines axialen Zurückziehens des Freigabegriffs (101) distal bewegt, wobei, wenn der Freigabegriff (101) gedreht wird, der Freigabegriff (101) in den Griffhalter (102) eingreift und in Bezug auf den Griffhalter (102) axial stationär bleibt,
**dadurch gekennzeichnet, dass**:
ein Freigabeschalter (105) zum Begrenzen einer axialen Bewegung und Umfangsdrehung des Freigabegriffs (101) zwischen dem Freigabegriff (101) und dem Griffhalter (102) angeordnet ist,
der Freigabeschalter (105) ein distales Ende aufweist, das mit einem Nocken (105a) versehen ist, und eine Innenfläche eines proximalen Endes des Freigabegriffs (101) mit einer Vielzahl von Begrenzungsnuten (101a) versehen ist, die in Bezug auf den Freigabegriff (101) in Umfangsrichtung angeordnet sind, wobei jede Begrenzungsnut (101a) mit dem Nocken (105a) in Eingriff gebracht werden kann und an beiden Enden entlang einer Umfangsrichtung Umfangsbegrenzungsvorsprünge aufweist.

2. Zuführungssystem für ein medizinisches Implantat nach Anspruch 1, wobei der Griffhalter (102) ein distales Ende aufweist, das mit einem ersten Spalt versehen ist, wobei der Freigabeschalter (105) teilweise in den ersten Spalt eingebettet ist und eine Achse des Freigabeschalters (105) parallel zu einer Achse des Griffgehäuses (11) ist.

3. Zuführungssystem für ein medizinisches Implantat nach Anspruch 2, wobei der Griffhalter (102) mit einer ringähnlichen Halterungsplatte (102b) versehen ist, die mit dem Freigabeschalter (105) zusammenpasst, wobei die ringähnliche Halterungsplatte (102b) eine Achse parallel zu der Achse des Griffgehäuses (11) aufweist, und wobei der Freigabeschalter (105) ein proximales Ende aufweist, das mit mindestens zwei Überbrückungsschenkeln (105c) versehen ist, wovon jeder mit einer Innenfläche der ringähnlichen Halterungsplatte (102b) in Eingriff ist.

4. Zuführungssystem für ein medizinisches Implantat nach Anspruch 3, wobei eine Außenfläche der ringähnlichen Halterungsplatte (102b) an einer ersten Kerbe und einer zweiten Kerbe auf eine Innenfläche des Griffhalters (102) trifft, wobei das proximale Ende des Freigabeschalters (105) mit einem Drehanschlag (105b) versehen ist, und wobei, wenn der Freigabeschalter (105) im Uhrzeigersinn oder gegen den Uhrzeigersinn gedreht wird, der Drehanschlag (105b) zu der ersten Kerbe oder der zweiten Kerbe gedreht wird, der Drehanschlag (105b) in die erste Kerbe oder die zweite Kerbe eingreift und somit einen maximal zulässigen Drehwinkel für den Freigabeschalter (105) definiert.

5. Zuführungssystem für ein medizinisches Implantat nach Anspruch 4, wobei der maximal zulässige Drehwinkel für den Freigabeschalter (105) kleiner als oder gleich wie 180° ist.

6. Zuführungssystem für ein medizinisches Implantat nach Anspruch 1, wobei der Nocken (105a) mit einem einseitigen Begrenzungsvorsprung versehen ist, wobei jede der Vielzahl von Begrenzungsnuten (101a) mit einer dritten Kerbe versehen ist, die mit dem einseitigen Begrenzungsvorsprung in Eingriff gebracht werden kann, wobei der einseitige Begrenzungsvorsprung und die dritte Kerbe zusammenwirken, um ein Drehen in eine Richtung für den Freigabeschalter (105) zu ermöglichen.

7. Zuführungssystem für ein medizinisches Implantat nach Anspruch 1, wobei das Griffgehäuse (11) eine obere Griffschale (103) und eine untere Griffschale (104, 604, 704) umfasst, die lösbar verbunden sind, und
wobei die obere Griffschale (103) darin mit einer oberen Griffschalenverstärkungsplatte (103a) entlang einer axialen Richtung der oberen Griffschale (103) versehen ist, wobei die untere Griffschale (104, 604, 704) darin mit einer unteren Griffschalenverstärkungsplatte (104a, 604a, 704a) entlang einer axialen Richtung der unteren Griffschale (104, 604, 704) versehen ist, wobei auf einer Seite der Zahnstange (20) nahe der unteren Griffschale (104, 604, 704) eine Zahnstangenführung (20c) bereitgestellt ist, die in der Lage ist, mit der unteren Griffschalen-Verstärkungsplatte (104a, 604a, 704a) zusammenzupassen, wobei sich die Zahnstangenführung (20c) entlang der axialen Richtung des Griffgehäuses (11) erstreckt, und wobei die Zahnstange (20) an der oberen Griffschalenverstärkungsplatte (103a) auf einer Seite davon nahe der oberen Griffschale (103) anliegt.

8. Zuführungssystem für ein medizinisches Implantat nach Anspruch 7, wobei auf der Zahnstange (20) eine Vielzahl von Zahnstangenverstärkungsrippen (20d) in Abständen entlang einer axialen Richtung der Zahnstange (20) bereitgestellt sind.

9. Zuführungssystem für ein medizinisches Implantat nach Anspruch 7, wobei die obere Griffschale (103) ferner mit einem wabenartigen Netz von Verstärkungsrippen (103b) der oberen Griffschale versehen ist und die untere Griffschale (104, 604, 704) ferner mit einem wabenartigen Netz von Verstärkungsrippen (104b) der unteren Griffschale versehen ist, und
wobei die oberen Griffschalenverstärkungsrippen (103b) an der oberen Griffschalenverstärkungsplatte (103a) angebracht sind und die unteren Griffschalenverstärkungsrippen (104b) an der unteren Griffschalenverstärkungsplatte (104a, 604a, 704a) angebracht sind.

10. Zuführungssystem für ein medizinisches Implantat nach einem der Ansprüche 1 bis 9, wobei das Griffgehäuse (11) einen Spitzenabschnitt (11b), einen ersten Schaftabschnitt (11c) und einen zweiten Schaftabschnitt (11d) umfasst, die in axialer Richtung in Reihe verbunden sind, wobei der Griffhalter (102) fest um eine Verbindungsstelle des ersten und zweiten Schaftabschnitts angeordnet ist.

11. Zuführungssystem für ein medizinisches Implantat nach Anspruch 10, wobei ein Durchmesser des ersten Schaftabschnitts (11c) größer ist als ein Durchmesser des zweiten Schaftabschnitts (11d), eine Absatz (11a) an der Verbindungsstelle des ersten und des zweiten Schaftabschnitts bereitgestellt ist und der Griffhalter (102) fest um den Absatz (11a) herum angeordnet ist, und/oder
wobei der Spitzenabschnitt (11b) einem Spitzenabschnitt eines Stiftes ähnelt und jeder von dem ersten und dem zweiten Schaftabschnitt (11c, 11d) ein hohles röhrenförmiges Element ist, das mit dem Schlitz in axialer Richtung versehen ist.

12. Zuführungssystem für ein medizinisches Implantat nach einem der Ansprüche 1 bis 9, wobei die Zahnstange (20) einen inneren Zahnstangenhohlraum (20b) definiert, der sich entlang der axialen Richtung durch die Zahnstange (20) erstreckt.

13. Zuführungssystem für ein medizinisches Implantat nach Anspruch 2, wobei der Griffhalter (102) einen zweiten Spalt definiert, der dem Schlitz entlang der axialen Richtung entspricht.

14. Zuführungssystem für ein medizinisches Implantat nach Anspruch 1, ferner umfassend eine äußere Hülle (304) mit einem distalen Ende, das mit einem proximalen Ende der Zahnstange (20) verbunden ist, wobei ein Drehen oder axiales Zurückziehen des Freigabegriffs (101) eine distale Bewegung der Zahnstange (20) und somit eine distale Bewegung der äußeren Hülle (304) mit dem Freigabegriff (101) bewirkt.

## Revendications

1. Système d'administration pour un implant médical, comprenant une coque de poignée (11), une poignée de libération (101), un support de poignée (102) et une crémaillère (20), la coque de poignée (11) mise en oeuvre sous la forme d'un élément tubulaire creux définissant une fente s'étendant le long d'une direction axiale de la coque de poignée (11), la crémaillère (20) reçue à l'intérieur de la coque de poignée (11) dans une orientation le long de la direction axiale de la coque de poignée (11), au moins certaines des dents de la crémaillère (20) faisant saillie hors de la fente, le support de poignée (102) disposé de manière fixe sur la coque de poignée (11), la poignée de libération (101) disposée sur la coque de poignée (11) en prise avec les dents de sorte que la crémaillère (20) se déplace de manière distale en conséquence d'une rotation ou d'une rétraction axiale de la poignée de libération (101), dans lequel lorsque la poignée de libération (101) est tournée, la poignée de libération (101) se met en prise avec le support de poignée (102) et reste stationnaire axialement par rapport au support de poignée (102),
**caractérisé en ce que** :
un commutateur de libération (105) destiné à limiter le mouvement axial et la rotation circonférentielle de la poignée de libération (101) est agencé entre la poignée de libération (101) et le support de poignée (102),
le commutateur de libération (105) a une extrémité distale pourvue d'une came (105a), et une surface interne d'une extrémité proximale de la poignée de libération (101) pourvue d'une pluralité de rainures de limitation (101a) agencées circonférentiellement par rapport à la poignée de libération (101), chaque rainure de limitation (101a) pouvant être mise en prise avec la came (105a) et ayant des saillies de limitation circonférentielles sur les deux extrémités le long d'une direction circonférentielle.

2. Système d'administration pour un implant médical selon la revendication 1, dans lequel le support de poignée (102) a une extrémité distale pourvue d'un premier espace, le commutateur de libération (105) partiellement encastré dans le premier espace et un axe du commutateur de libération (105) étant parallèle à un axe de la coque de poignée (11).

3. Système d'administration pour un implant médical selon la revendication 2, dans lequel le support de poignée (102) est pourvu d'une plaque de support en forme d'anneau (102b) adaptée au commutateur de libération (105), la plaque de support en forme d'anneau (102b) ayant un axe parallèle à l'axe de la coque de poignée (11), et dans lequel le commutateur de libération (105) a une extrémité proximale pourvue d'au moins deux pattes de pontage (105c), chacune d'entre elles venant en prise avec une surface interne de la plaque de support en forme d'anneau (102b).

4. Système d'administration pour un implant médical selon la revendication 3, dans lequel une surface externe de la plaque de support en forme d'anneau (102b) rencontre une surface interne du support de poignée (102) au niveau d'une première encoche et d'une seconde encoche, dans lequel l'extrémité proximale du commutateur de libération (105) est pourvue d'une butée de rotation (105b), et dans lequel, lorsque l'interrupteur de libération (105) est tourné dans le sens des aiguilles d'une montre ou dans le sens inverse, la butée de rotation (105b) est tournée vers la première encoche ou la seconde encoche, la butée de rotation (105b) est en prise avec la première encoche ou la seconde encoche, définissant ainsi un angle de rotation maximal admissible pour le commutateur de libération (105).

5. Système d'administration pour un implant médical selon la revendication 4, dans lequel l'angle de rotation maximal admissible pour le commutateur de libération (105) est inférieur ou égal à 180°.

6. Système d'administration pour un implant médical selon la revendication 1, dans lequel la came (105a) est pourvue d'une saillie de limitation unilatérale, chacune de la pluralité de rainures de limitation (101a) étant pourvue d'une troisième encoche pouvant être mise en prise avec la saillie de limitation unilatérale, la saillie de limitation unilatérale et la troisième encoche coopèrent pour permettre une rotation unidirectionnelle pour le commutateur de libération (105).

7. Système d'administration pour un implant médical selon la revendication 1, dans lequel la coque de poignée (11) comprend une coque de poignée supérieure (103) et une coque de poignée inférieure (104, 604, 704) qui sont connectées de manière détachable, et
dans lequel la coque de poignée supérieure (103) est pourvue à l'intérieur d'une plaque de renforcement de coque de poignée supérieure (103a) le long d'une direction axiale de la coque de poignée supérieure (103), dans lequel la coque de poignée inférieure (104, 604, 704) est pourvue à l'intérieur d'une plaque de renforcement de coque de poignée inférieure (104a, 604a, 704a) le long d'une direction axiale de la coque de poignée inférieure (104, 604, 704), dans lequel, sur un côté de la crémaillère (20) proche de la coque de poignée inférieure (104, 604, 704), est prévue une glissière de crémaillère (20c) capable de s'adapter à la plaque de renforcement de coque de poignée inférieure (104a, 604a, 704a), la glissière de crémaillère (20c) s'étendant le long de la direction axiale de la coque de poignée (11), et dans lequel la crémaillère (20) bute contre la plaque de renforcement de coque de poignée supérieure (103a) sur un côté de celle-ci proche de la coque de poignée supérieure (103).

8. Système d'administration pour un implant médical selon la revendication 7, dans lequel une pluralité de nervures de renforcement de crémaillère (20d) sont prévues sur la crémaillère (20) à des intervalles le long d'une direction axiale de la crémaillère (20).

9. Système d'administration pour un implant médical selon la revendication 7, dans lequel la coque de poignée supérieure (103) est en outre pourvue à l'intérieur d'un réseau de type nid d'abeille de nervures de renforcement de la coque de poignée supérieure (103b), et la coque de poignée inférieure (104, 604, 704) est en outre pourvue à l'intérieur d'un réseau de type nid d'abeille de nervures de renforcement de la coque de poignée inférieure (104b), et
dans lequel les nervures de renforcement de la coque de poignée supérieure (103b) sont fixées à la plaque de renforcement de la coque de poignée supérieure (103a), et les nervures de renforcement de la coque de poignée inférieure (104b) sont fixées à la plaque de renforcement de la coque de poignée inférieure (104a, 604a, 704a).

10. Système d'administration pour un implant médical selon l'une quelconque des revendications 1 à 9, dans lequel la coque de poignée (11) comprend une section de pointe (11b), une première section de tige (11c) et une seconde section de tige (11d), qui sont connectées en série dans la direction axiale, dans lequel le support de poignée (102) est disposé de manière fixe autour d'une jonction des première et seconde sections de tige.

11. Système d'administration pour un implant médical selon la revendication 10, dans lequel un diamètre de la première section de tige (11c) est supérieur à un diamètre de la seconde section de tige (11d), un épaulement (11a) prévu à la jonction des première et seconde sections de tige, et le support de poignée (102) disposé de manière fixe autour de l'épaulement (11a), et/ou
dans lequel la section de pointe (11b) ressemble à une partie de pointe d'un stylo, et chacune des première et seconde sections de tige (11c, 11d) est un élément tubulaire creux pourvu de la fente dans la direction axiale.

12. Système d'administration pour un implant médical selon l'une quelconque des revendications 1 à 9, dans lequel la crémaillère (20) définit une cavité interne de crémaillère (20b) s'étendant à travers la crémaillère (20) le long de la direction axiale.

13. Système d'administration pour un implant médical selon la revendication 2, dans lequel le support de poignée (102) définit un second espace correspondant à la fente le long de la direction axiale.

14. Système d'administration pour un implant médical selon la revendication 1, comprenant en outre une gaine externe (304) avec une extrémité distale connectée à une extrémité proximale de la crémaillère (20), dans lequel la rotation ou la rétraction axiale de la poignée de libération (101) provoque un mouvement distal de la crémaillère (20) et donc un mouvement distal de la gaine externe (304) avec la poignée de libération (101).
